# FASCICULE DE BREVET EUROPEEN

(11) **EP 0 402 251 B1**
(45) Date de publication et mention de la délivrance du brevet: **24.08.1994**
(21) Numéro de dépôt: 90401540.1
(22) Date de dépôt: 06.06.1990
(51) Int. Cl.: A61F 11/04, A61F 2/02, A61N 1/36

(54) **Dispositif de connexion transcutanée**
Transkutane Verbindungseinrichtung
Transcutaneous connection device

(30) Priorité: 07.06.1989 FR 8907557; 02.04.1990 FR 9004191
(43) Date de publication de la demande: 12.12.1990
(73) Titulaire: ASSISTANCE PUBLIQUE, 75100 Paris (FR)
(72) Inventeur: Frachet, Bruno, F-95160 Montmorency (FR); David, Michel Yves, F-75014 Paris (FR)
(74) Mandataire: Le Guen, Gérard

(56) Documents cités:
- FR-A- 2 220 231
- US-A- 4 063 048
- US-A- 4 617 913
- US-A- 4 645 504
- ELECTRONIQUE APPLICATIONS

## Description

La présente invention concerne un dispositif de connexion transcutanée servant à relier un appareil de stimulation bioélectrique extérieur à l'organisme à un ou plusieurs conducteurs électriques sous-cutanés, et plus particulièrement, à une électrode destinée à une stimulation électrique de l'oreille interne dans le cas de surdité neurosensorielle complète ou d'acouphènes (see par exemple US-A-4 617 913).

L'emploi de stimulations électriques est large, qu'il s'agisse de stimulation musculaire ou nerveuse. L'excitation électrique peut être délivrée indirectement ou directement :
- indirectement, au travers de la peau qui reste intacte : en utilisant un dispositif avec deux bobines d'induction soumises à un courant haute fréquence. (technique classique d'implantation).
- directement, par un conducteur métallique qui traverse la peau. Ce mode d'excitation directe simplifie le système générateur mais expose à des complications cutanées, essentiellement d'ordre infectieux.

La présente invention repose sur l'idée consistant à utiliser le pavillon de l'oreille pour réaliser un dispositif de connexion directe transcutanée.

La présente invention a donc pour objet un dispositif de connexion transcutanée destiné à relier un stimulateur électrique extérieur à l'organisme à un conducteur électrique sous-cutané, relié à une électrode, caractérise en ce qu'il est fixé dans le pavillon de l'oreille et comprend au moins un fil conducteur sous-cutané (3, 3a) revêtu d'une gaine isolante, dont une extrémité distale est connectée audit conducteur électrique sous-cutané, des moyens de connexion extérieurs audit pavillon de l'oreille (2,4-2a,30) pour la connexion de l'extrémité proximale dudit ou desdits fils(s) conducteurs(s) sous-cutané(s) audit stimulateur électrique extérieur et une électrode de masse en contact avec la peau.

Dans un premier mode de réalisation de l'invention, le dispositif placé dans le pavillon de l'oreille est positionné dans un repli du pavillon situé sous la fossette naviculaire et comprend avantageusement au moins un fil conducteur sous-cutané revêtu d'une gaine isolante, fixé à une sphère en métal conducteur positionnée à la surface du pavillon de l'oreille et revêtue d'un matériau isolant sur la partie de sa surface externe au contact du pavillon de l'oreille, le fil conducteur et la sphère consistant de préférence en platine-iridium, le matériau et la gaine isolants consistant avantageusement en un matériau isolant biocompatible, de préférence Téflon et Parylène®.

Dans un second mode de réalisation, le dispositif placé dans le pavillon de l'oreille comprend une boucle d'oreille positionnée dans le lobule de l'oreille.

Avantageusement la boucle d'oreille comporte d'une part une tige connectée à un ensemble sous-cutané comprenant au moins un fil conducteur dont l'extrémité distale est reliée au conducteur électrique sous-cutané et l'extrémité proximale à ladite tige, et d'autre part un fermoir connecté au stimulateur électrique extérieur à l'organisme.

La tige de la boucle d'oreille est de manière avantageuse isolée au moyen d'une gaine plastique biocompatible.

De préférence dans les deux modes de réalisation de l'invention, ledit fil conducteur est torsadé, par exemple de manière à former ressort et consiste en un matériau biocompatible, qui est avantageusement du Phynox®, le fil conducteur torsadé étant recouvert par un matériau isolant biocompatible, de préférence du Parylène®.

Suivant une autre variante de réalisation préférée de l'invention, le dispositif de connexion transcutané comprend trois fils conducteurs sous-cutanés torsadés de manière à constituer trois ressorts hélicoïdaux imbriqués, ou comprend trois fils conducteurs tressés ensemble.

Le fil conducteur assurant la connexion entre le conducteur électrique sous-cutané et la tige de la boucle d'oreille ou la sphère placée sous la fossette naviculaire peut être relié au conducteur électrique sous-cutané par tout moyen approprié.

Un premier moyen approprié consiste en ce que le conducteur électrique sous-cutané et le fil conducteur relié à la sphère conductrice ou la boucle d'oreille sont reliés l'un à l'autre par soudure.

Un second moyen approprié est constitué par une épissure réalisée par exemple dans la mastoîde par une intervention chirurgicale.

Un troisième moyen approprié consiste en au moins deux connecteurs électriques, chaque connecteur consistant avantageusement en une pièce composite qui comporte une partie mâle formant fiche et une partie femelle formant douille dans laquelle vient s'emboîter la partie mâle formant fiche de l'autre connecteur.

La sphère conductrice ou la boucle d'oreille sont reliées au stimulateur extérieur par tout moyen approprié. On préfère toutefois des moyens qui sont amovibles. Dans le cas de la sphère conductrice, ceux-ci consistent de préférence en un embout métallique ou métallisé épousant les reliefs du pavillon de l'oreille.

De manière avantageuse, la sphère se positionne par encliquetage dans une alvéole aménagée dans l'embout, de forme et de dimensions correspondant sensiblement à celles de la sphère, l'alvéole étant isolée de l'embout et sa surface étant connectée au stimulateur extérieur et constituant le pôle actif, la masse métallique ou la surface métallisée de l'embout assurant un contact de masse avec la peau du pavillon et du conduit auditif.

Dans le cas de la boucle d'oreille, la liaison amovible entre l'appareil de stimulation et la boucle d'oreille est avantageusement réalisée au moyen d'un aimant, de préférence de forme cylindrique, fixé sur la boucle d'oreille sur lequel s'applique une masse magnétique consistant préférentiellement en un second aimant de même forme relié à la sortie du stimulateur.

Avantageusement, le premier aimant est fixé dans l'une des branches du fermoir de la boucle d'oreille au moyen d'une gaine métallique conductrice fixée dans la branche du fermoir par soudure de façon à ce que le bord de la gaine métallique conductrice dépasse par rapport audit aimant de manière à accueillir le second aimant relié à la sortie du stimulateur extérieur, les deux aimants étant orientés pour une attraction réciproque lors de la connexion.

D'autres caractéristiques et avantages de l'invention apparaîtront au cours de la description détaillée qui va suivre en regard des dessins annexés, sur lesquels :
- la Fig. 1 représente une vue du pavillon de l'oreille indiquant la position du dispositif de connexion transcutanée selon le premier mode de réalisation de l'invention;
- la Fig. 2 est une vue de l'embout assurant la liaison électrique entre le dispositif de connexion transcutanée selon le premier mode de réalisation de l'invention et le stimulateur extérieur;
- la Fig. 3 est une vue en coupe longitudinale de l'embout représenté à la Fig. 2 connecté avec la sphère selon le premier mode de réalisation de l'invention;
- la Fig. 4 représente une vue en coupe longitudinale du lobule de l'oreille, équipé du dispositif selon le second mode de réalisation de l'invention;
- la Fig. 5 est une vue en perspective éclatée, avec arrachement partiel, du dispositif selon le second mode de réalisation de l'invention, avec ses moyens de connexion au stimulateur externe, dans laquelle le dispositif est déconnecté du stimulateur extérieur;
- la Fig. 6 représente une vue en coupe des moyens de connexion reliant le dispositif de la Fig. 5 au stimulateur externe ;
- la figure 7 est une vue de profil d'une variante de réalisation des moyens de connexion au stimulateur externe ;
- la figure 8 est une vue en coupe axiale du connecteur mâle des moyens de connexion de la figure 7;
- la figure 9 est une vue de droite correspondant à la figure 8 ;
- la figure 10 est une vue en coupe axiale de l'extrémité du connecteur femelle des moyens de connexion de la figure 7 ;
- la figure 11 est une vue de gauche correspondant à la figure 10.

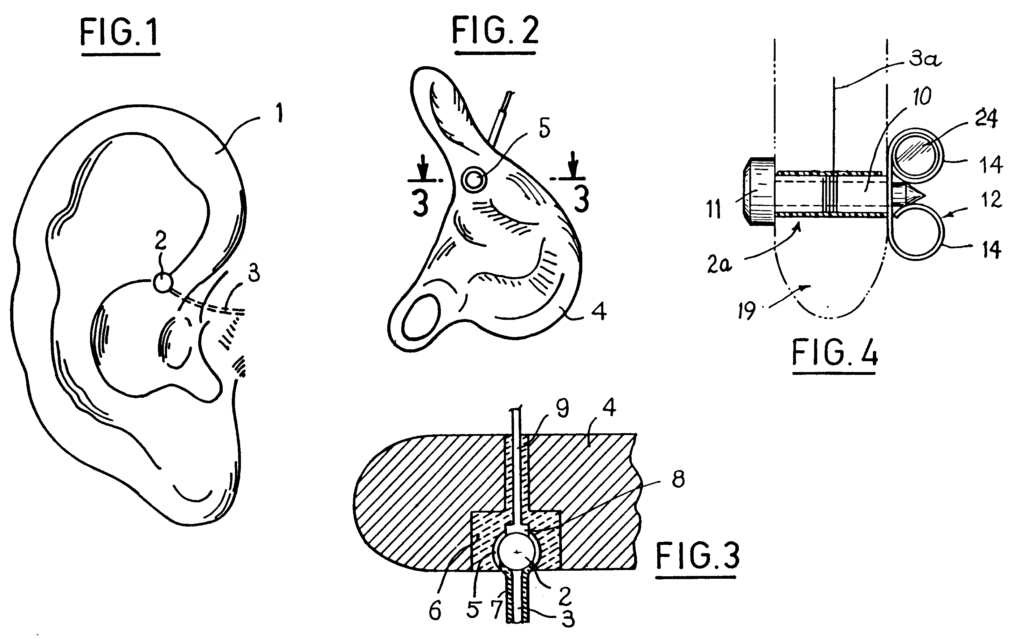

Dans le premier mode de réalisation de l'invention, le dispositif est fixé au niveau du pavillon de l'oreille 1 dans un repli de celui-ci placé sous la fossette naviculaire comme illustré sur la fig. 1 et comporte une sphère 2 située en surface du pavillon de l'oreille reliée à un fil conducteur 3 sous-cutané, avantageusement par soudure.

La sphère 2 a avantageusement un diamètre d'environ 3 mm et consiste comme le fil conducteur 3 en un alliage platine-iridium.

Le fil conducteur 3 et la surface de la sphère 2 en contact avec la peau du pavillon de l'oreille sont isolées au moyen d'un revêtement qui est avantageusement constitué de Téflon et de Parylène®. Le fil conducteur 3 perfore la surface cutanée et se dirige vers la connexion avec le conducteur électrique sous-cutané consistant par exemple en une électrode de stimulation placée au niveau de l'oreille interne.

La sphère 2 est reliée au stimulateur extérieur non représenté au moyen d'un embout 4 métallique ou métallisé épousant les reliefs du pavillon de l'oreille comme représenté sur la fig. 2.

L'embout 4 est avantageusement en or et est confectionné à partir d'une empreinte réalisée par moulage et reproduisant avec le plus de précision possible les reliefs du pavillon de l'oreille en particulier ceux de la conque et de la fossette sous naviculaire.

L'embout 4 comporte une alvéole 5 de forme et de dimensions correspondant à celles de la sphère 2.

L'embout 4 relié au stimulateur extérieur est placé au contact du pavillon de l'oreille, sa face antérieure visible sur la fig. 2 venant s'appliquer au contact de la peau du pavillon de telle manière que la sphère 2 soit en regard de l'alvéole 5 et vienne s'encliqueter dans celle-ci comme représenté sur la fig. 3.

L'alvéole 5 est isolée de l'embout 4 au moyen d'une gaine isolante 6. La sphère 2 est fixée au fil conducteur 3 entouré d'une gaine isolante 7, celle-ci venant coiffer la surface extérieure de la sphère 2 pour isoler cette sphère de la peau du pavillon de l'oreille. Lorsqu'elle est positionnée dans l'alvéole 5 par encliquetage, la sphère 2 vient s'appliquer au contact de l'extrémité aplatie 8 du fil 9 relié au stimulateur extérieur, l'alvéole 5 formant ainsi le pôle actif, l'embout métallique 4 assurant un contact de masse avec la peau du pavillon de l'oreille et du conduit auditif.

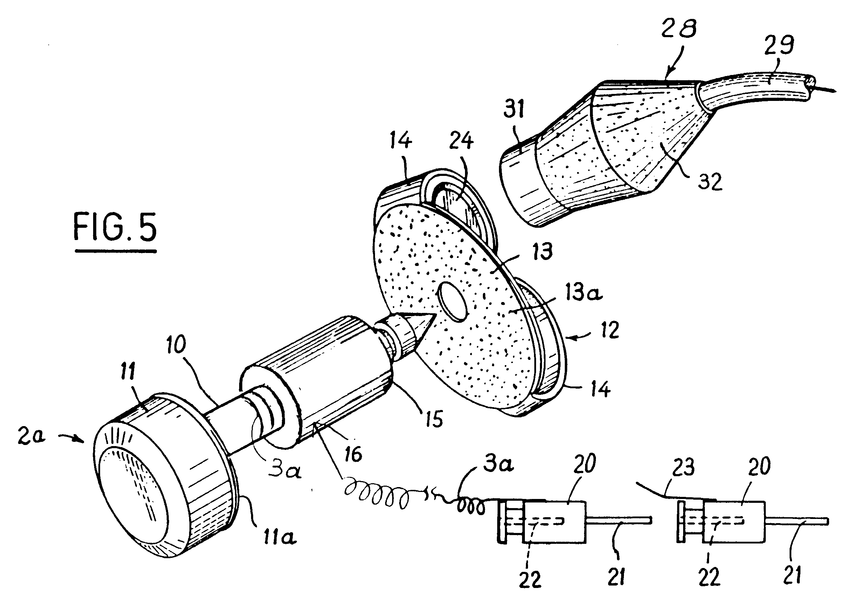

Dans le second mode de réalisation de l'invention, le dispositif est fixé au niveau du lobule 19 de l'oreille comme illustré sur les figures 4 et 5. Il comporte une boucle d'oreille 2a fixée dans le lobule 19 de l'oreille. La boucle d'oreille 2a comprend une tige 10 traversant le lobule de l'oreille et comporte à son extrémité antérieure un motif 11 et à son extrémité postérieure un fermoir 12 qui comprend un disque central 13 perforé pour laisser passer la tige 10 et deux branches 14 recourbées sur elles-mêmes pour former chacune un tube.

Avantageusement, le disque central 13 et la face interne du motif 11 sont isolés du lobe de l'oreille respectivement au moyen d'un disque isolant 13a et d'une pastille isolante 11a.

La tige 10 de la boucle d'oreille est reliée à un fil 3a torsadé formant ressort composé matériau électro-conducteur tel que le Phynox® dans le lobule de l'oreille par intervention chirurgicale, le fil torsadé étant lui-même relié à un conducteur électrique sous-cutané.

Le fil 3a est isolé sur son parcours sous-cutané au moyen d'une gaine formée d'un matériau isolant biocompatible tel que le Parylène®.

L'extrémité distale du fil 3a est connectée au conducteur électrique sous-cutané, qui peut consister en un fil relié à une électrode de stimulation telle que celle décrite dans le brevet FR-2 613 575, utilisée dans le cas de surdité complète ou dans le traitement des acouphènes.

Le conducteur électrique sous-cutané peut également consister en tout conducteur électrique unique ou multiple destiné à la stimulation d'une structure nerveuse ou musculaire.

Dans le cas où le dispositif selon l'invention est destiné à relier une électrode appliquée sur la fenêtre ovale au niveau de l'oreille interne, la pose de l'électrode de stimulation et du dispositif de connexion transcutanée est réalisée au cours de la même intervention chirurgicale. Le fil 3a est connecté au conducteur sous-cutané, par exemple au fil aboutissant à l'électrode de stimulation au moyen d'une soudure, d'une épissure réalisée dans la mastoïde au cours de l'intervention chirurgicale ou encore àu moyen de petits connecteurs électriques.

L'extrémité proximale du fil 3a est enroulée autour de la tige 10 de la boucle d'oreille. La tige 10 de la boucle d'oreille 2a et le fil 3a enroulé sur celle-ci sont isolés au moyen d'une gaine plastique isolante 15 qui consiste de préférence en une portion de cathéter, le fil 3a perforant cette gaine isolante au niveau 16 situé avantageusement au milieu de la tige 10.

Le fil 3a peut également être coudé et simplement glissé sous la gaine isolante 15.

La fig. 5 représente en outre deux connecteurs électriques 20 destinés à assurer un autre type de connexion entre le fil 3a et le conducteur électrique sous-cutané que la soudure ou l'épissure.

Chaque connecteur 20 consiste en une pièce composite comportant une partie mâle formant fiche 21 et une partie femelle formant douille 22.

L'un des connecteurs 20 est relié au fil conducteur 3a au moyen par exemple d'une soudure. La partie mâle formant fiche 21 de ce premier connecteur peut alors s'emboîter dans la partie femelle formant douille 22 d'un second connecteur 20 relié au conducteur électrique sous-cutané 23, lequel est relié par exemple à l'électrode de stimulation de l'oreille interne précédemment décrite.

La connexion est mise en place par le chirurgien au moment de la pose du système de connexion transcutané selon l'invention. Elle peut être constituée par un nombre de connecteurs supérieur à deux empilés les uns dans les autres par leurs parties mâle et femelle et reliés à autant de conducteurs sous-cutanés.

Ce système de connexion au moyen de petits connecteurs empilés peut bien évidemment être utilisé dans tous les modes de réalisatiuon du dispositif transcutané de l'invention incluant le dispositif constitué par la sphère 2 et le fil 3.

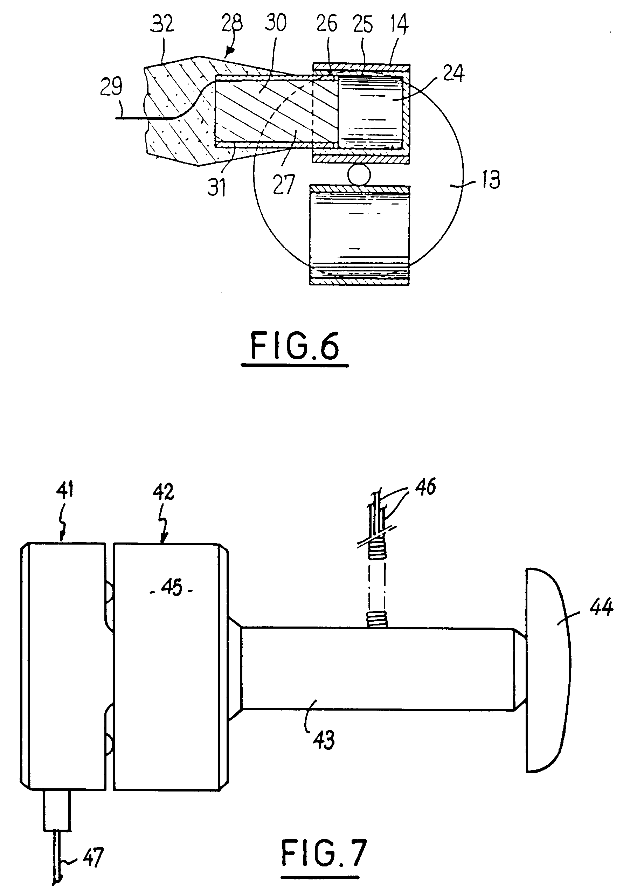

La figure 6 représente une vue en coupe du système de connexion du dispositif du deuxième mode de réalisation selon l'invention, au stimulateur bioélectrique.

Dans l'une des branches 14 du fermoir 12 est fixé un petit aimant 24 au moyen d'une gaine métallique conductrice 25 soudée dans la branche du fermoir, l'aimant étant avantageusement de forme cylindrique et l'ensemble étant de diamètre sensiblement égal à celui formé par la repliement de la branche 14. Avantageusement, la longueur de l'aimant 24 est plus courte que la longueur de la gaine 25 de manière à ce que le bord 26 de la gaine métallique électroconductrice dépasse par rapport à l'aimant 24 et forme un espace en forme de cuvette 27 qui permet à un second aimant 30 de s'encastrer dans la cuvette 27, les deux aimants 24 et 30 étant par ailleurs orientés par une attraction réciproque de manière à assurer un contact électrique optimal.

Un connecteur 28 est prévu à l'extrémité du fil 29 sortant de l'appareil de stimulation et comporte une petite masse magnétique qui est avantageusement le petit aimant 30 ayant également la forme d'un cylindre dont le diamètre est légèrement inférieur à celui de l'aimant 24. Cet aimant 30 est recouvert sur sa surface périphérique externe d'une gaine 31 en métal électroconducteur.

La gaine 31 est destinée à relier le fil conducteur 29 à l'aimant 30 et elle assure en outre la conduction électrique entre le stimulateur bioélectrique et la branche 14 du fermoir de la boucle d'oreille 2a laquelle est assurée également au moyen de l'aimant 30 suffisamment conducteur électriquement. La connexion du fil 29 sortant de l'appareil de stimulation avec l'aimant 30 est protégée au moyen d'une gaine 32 formée de résine polymère. Le courant électrique est ensuite transmis par le biais de la tige 10 au fil 3a.

Le fil 3a transmet alors les implusions électriques au conducteur électrique sous-cutané, par exemple par l'intermédiaire d'une soudure, d'une épissure non représentée réalisée au niveau de la mastoïde, ou par l'intermédiaire des petits connecteurs électriques représentés sur la fig. 5, à un fil relié à une électrode de stimulation électrique appliquée sur la membrane de la fenêtre ovale.

Dans la description du second mode de réalisation de l'invention, un seul pôle est pris en considération. L'autre, la masse, est constitué par une électrode de référence qui est, par exemple, dans le cas d'un dispositif reliant une électrode de stimulation placée au niveau de l'oreille interne, une large électrode moulée placée dans la conque de l'oreille du type de l'embout précédemment décrit qui constitue ainsi une masse cutanée de bonne surface. Toute autre électrode de masse est cependant envisageable. En particulier, la boucle d'oreille peut donner lieu à plusieurs connexions, dont une de masse.

Le conducteur électrique sous-cutané, peut consister en un fil relié à une électrode de stimulation telle que celle décrite dans le brevet FR 2 613 575, utilisée dans le cas de surdité complète ou dans le traitement des acouphènes.

Le conducteur électrique sous-cutané peut également consister en tout conducteur électrique unique ou multiple destiné à la stimulation d'une structure nerveuse ou musculaire.

Dans le cas où le dispositif selon l'invention est destiné à relier une électrode appliquée sur la fenêtre ovale au niveau de l'oreille interne, la pose de l'électrode de stimulation et du dispositif de connexion transcutanée est réalisée au cours de la même intervention chirurgicale.

Comme indiqué plus haut, on peut prévoir plusieurs fils de conducteur sous-cutanés ; dans ce cas il faut également prévoir un nombre égal de conducteurs extérieurs réalisant la liaison avec le stimulateur extérieur. On peut par exemple prévoir une liaison à trois conducteurs et les figures 7 à 11 illustrent des moyens de connexion entre trois fils sous-cutanés et trois conducteurs extérieurs.

Ces moyens de connexion sont constitués par un connecteur mâle 41 et un connecteur femelle 42. Le connecteur femelle comporte une tige 43 qui traverse le pavillon de l'oreille et qui comporte à une de ses extrémités un bouton 44 qui vient s'appliquer sur le pavillon de l'oreille et à son autre extrémité le connecteur mâle proprement dit 45.

Sur la figure 7 on a représenté les trois conducteurs sous-cutanés 46 qui sont enroulés les uns sur les autres de manière à réaliser trois ressorts hélicoïdaux imbriqués. Les trois conducteurs de sortie 47 sortent latéralement de l'élément mâle 41.

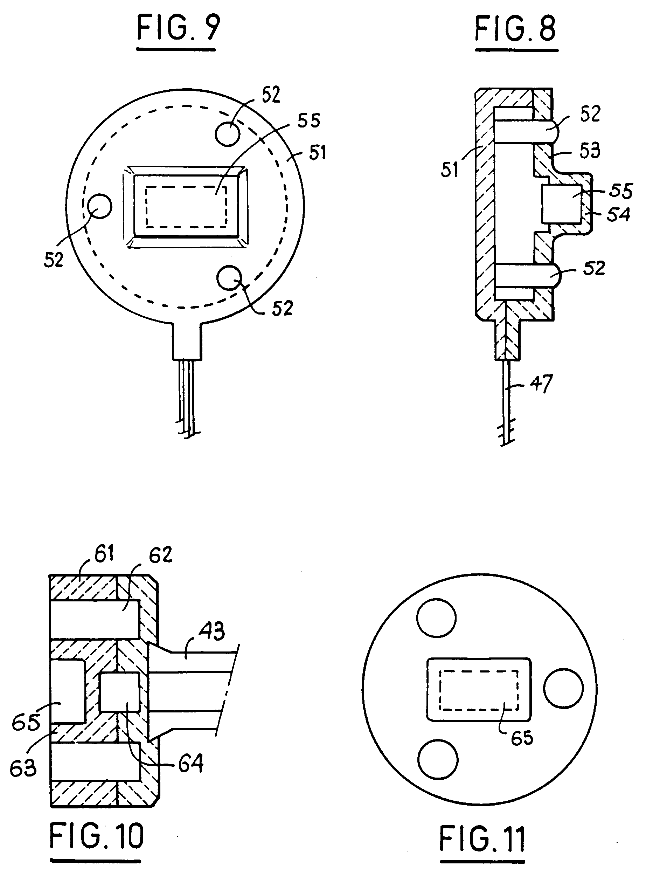

Les figure 8 et 9 représentent en détail le connecteur mâle. Il est constitué d'un boîtier circulaire 51 dans lequel sont disposées trois broches conductrices 52 selon un espacement angulaire régulier; ces trois broches font saillie d'une des faces 53 du boîtier 51. Cette face comporte également une partie centrale rectangulaire 54 faisant également saillie et constituant un logement pour un aimant 55. Les extrémités libres des broches 52 sont de forme arrondie.

Les figures 10 et 11 représentent la partie fonctionnelle du connecteur femelle 45 ; il comporte un boîtier 61 dans lequel sont logées des broches 62 qui sont disposées suivant une disposition analogue à celle des broches 52 du connecteur mâle. Une extrémité de chacune des broches 62 traverse une des faces 63 du boîtier 61, la surface extérieure libre des broches 62 se trouvant à effleurement avec cette face 63. Le boîtier 61 comporte un logement intérieur axial dans lequel est logé un aimant 64.

La face 63 comprend un évidement 65 de forme rectangulaire correspondant à la partie saillante 54 du connecteur mâle. Ces deux éléments 54 et 65 constituent un détrompeur assurant la connexion correcte des plots 52 sur les plots 62. Comme dans les autres modes de réalisation, la pression de contact est assurée par les forces magnétiques crées par les deux aimants 55 et 64.

Avantageusement, tous les éléments conducteurs sont réalisés avec le même matériau et reliés les uns aux autres par épissure ou par soudure autogène de manière à éviter les effets indésirables des couples électrochimiques créés par la jonction de deux matériaux différents.

Le dispositif de connexion transcutanée, objet de l'invention est d'une grande fiabilité et d'une manipulation aisée, notamment en ce qu'il est connecté à l'appareil de stimulation bioélectrique extérieur à l'organisme au moyen d'une connexion amovible et qu'un simple geste de la main suffit à le déconnecter.

Il trouve son application dans le domaine des surdités complètes ou des acouphènes, mais également dans le traitement de troubles non auditifs tels que la stimulation des structures nerveuses ou musculaires par exemple la stimulation de moignons ou le traitement électrique de douleurs localisées.

## Revendications

1. Dispositif de connexion transcutanée, destiné à relier un stimulateur électrique extérieur à l'organisme à un conducteur électrique sous-cutané relié à une électrode, caractérisé en ce qu'il est fixé dans le pavillon de l'oreille et comprend au moins un fil conducteur sous-cutané (3, 3a) revêtu d'une gaine isolante, dont une extrémité distale est connectée audit conducteur électrique sous-cutané, des moyens de connexion extérieurs audit pavillon de l'oreille (2, 4 - 2a, 30) pour la connexion de l'extrémité proximale dudit ou desdits fil(s) conducteur(s) sous-cutané(s) audit stimulateur électrique extérieur et une électrode de masse en contact avec la peau.

2. Dispositif de connexion transcutanée selon la revendication 1, caractérisé en ce que lesdits moyens de connexion extérieurs comprennent des moyens extérieurs (2, 2a) reliés audit ou auxdits fil(s) conducteur(s) sous-cutané (3, 3a) et des moyens extérieurs (4, 30) amovibles reliés audit stimulateur électrique extérieur.

3. Dispositif de connexion transcutanée selon la revendication 1 ou 2, caractérisé en ce qu'il est placé sous la fossette naviculaire dans un repli du pavillon de l'oreille.

4. Dispositif de connexion transcutanée selon la revendication 3, caractérisé en ce qu'il comprend un fil conducteur sous-cutané (3) revêtu d'une gaine isolante, fixé à une sphère (2) en métal conducteur positionnée à la surface du pavillon de l'oreille et revêtue d'un matériau isolant sur la partie de sa surface externe au contact du pavillon de l'oreille.

5. Dispositif de connexion transcutanée selon la revendication 4, caractérisé en ce que le fil conducteur sous-cutané (3) et la sphère (2) sont en platine-irridium.

6. Dispositif de connexion transcutanée selon la revendication 4 ou 5, caractérisé en ce que le fil conducteur sous-cutané (3) et la surface de la sphère (2) au contact du a pavillon de l'oreille sont isolés au moyen de Teflon® et de Parylène®.

7. Dispositif de connexion transcutanée selon l'une quelconque des revendications précédentes, caractérisé en ce que la sphère (2) est reliée au stimulateur extérieur au moyen d'un embout amovible (4) métallique ou métallisé épousant les reliefs du pavillon de l'oreille.

8. Dispositif de connexion transcutanée selon la revendication 7, caractérisé en ce que la sphère (2) se positionne par encliquetage dans une alvéole (5) aménagée dans l'embout amovible (4), de forme et de dimensions correspondant sensiblement à celles de la sphère (2), l'alvéole étant isolée de l'embout.

9. Dispositif de connexion transcutanée selon la revendication 8, caractérisé en ce que la surface de l'alvéole (5) est connectée au stimulateur extérieur et constitue le pôle actif, la masse métallique ou la surface métallisée de l'embout amovible (4) assurant un contact de masse avec la peau du pavillon et du conduit auditif.

10. Dispositif de connexion transcutanée selon la revendication 1, caractérisé en ce qu'il comprend une boucle d'oreille (2a) comportant d'une part une tige (10) connectée à un ensemble sous-cutané comprenant au moins un fil conducteur (3a) dont l'extrémité distale est reliée au conducteur électrique sous-cutané et l'extrémité proximale à ladite tige (10) et d'autre part, un fermoir (12) connecté au stimulateur électrique extérieur à l'organisme, et en ce que le fil conducteur (3a) consiste en un matériau biocompatible.

11. Dispositif de connexion transcutanée selon la revendication 4 ou 10, caractérisé en ce que le fil conducteur (3,3a) est torsadé.

12. Dispositif de connexion transcutanée selon la revendication 11, caractérisé en ce que le fil conducteur (3,3a) forme ressort.

13. Dispositif de connexion transcutanée selon la revendication 12, caractérisé en ce que le fil conducteur (3,3a) est en Phynox®.

14. Dispositif de connexion transcutanée selon la revendication 13, caractérisé en ce que le fil (3,3a) est recouvert par un matériau isolant de préférence du Parylène®.

15. Dispositif de connexion transcutanée selon la revendication 1, caractérisé en ce qu'il comprend trois fils conducteurs sous-cutanés (3,3a) torsadés et constituant trois ressorts hélicoïdaux imbriqués les uns dans les autres.

16. Dispositif de connexion transcutanée selon la revendication 1, caractérisé en ce qu'il comprend trois fils conducteurs sous-cutanés (3,3a) tressés ensemble.

17. Dispositif de connexion transcutanée selon la revendication 14, caractérisé en ce que la tige (10) de la boucle d'oreille (2a) est isolée au moyen d'une gaine plastique (16) biocompatible.

18. Dispositif de connexion transcutanée selon l' une quelconque des revendications 4 à 17, caractérisé en ce que le fil conducteur (3,3a) est relié au conducteur électrique sous-cutané au moyen d'au moins deux connecteurs électriques (20).

19. Dispositif de connexion transcutanée selon la revendication 18, caractérisé en ce que chaque connecteur électrique (20) consiste en une pièce composite comportant une partie mâle formant fiche (21) et une partie femelle (22) formant douille dans laquelle vient s'emboîter la partie mâle (21) formant fiche de l'autre connecteur (20).

20. Dispositif de connexion transcutanée selon la revendication 10, caractérisé en ce que la boucle d'oreille (2a) est reliée au stimulateur extérieur au moyen d'un aimant (24).

21. Dispositif de connexion transcutanée selon la revendication 20, caractérisé en ce que la boucle d'oreille est reliée au stimulateur extérieur au moyen d'un aimant de forme cylindrique fixé sur la boucle d'oreille sur lequel s'applique une masse magnétique amovible (30) de même forme reliée à la sortie du stimulateur.

22. Dispositif de connexion transcutanée selon la revendication 21, caractérisé en ce que la masse magnétique amovible (30) consiste en un second aimant.

23. Dispositif de connexion transcutanée l'oreille selon la revendication 22, caractérisé en ce que l'aimant (24) est fixé dans l'une des branches (14) du fermoir de la boucle d'oreille au moyen d'une gaine métallique (25) conductrice fixée dans la branche du fermoir par soudure de façon à ce que le bord (26) de ladite gaine métallique conductrice dépasse par rapport audit aimant (24) de manière à accueillir l'aimant (30), relié à la sortie du stimulateur extérieur, les deux aimants étant orientés pour une attraction réciproque lors de la connexion.

24. Dispositif de connexion transcutanée selon l'une quelconque des revendications précédentes, caractérisé en ce qu'il comprend plusieurs fils conducteurs sous-cutanés (46) et en ce que la connexion des fils sous-cutanés avec le stimulateur extérieur est réalisé au moyen d'un ensemble de connexion comportant un connecteur mâle (41) et un conducteur femelle (42) maintenus en position d'insertion au moyen d'au moins un aimant (55,64), lesdits connecteurs mâle (41) et femelle (42) comportant des éléments détrompeurs respectifs (54,65).

## Claims

1. Transcutaneous connecting device intended to connect an electrical stimulator outside the body to a subcutaneous electrical conductor connected to an electrode, characterised in that it is fixed in the ear pinna and comprises at least one subcutaneous conducting wire (3,3a) covered with an insulating sheath, a distal end of which is connected to said subcutaneous electrical conductor, connecting means outside the ear pinna (2,4-2a,30) for connecting the proximal end of said subcutaneous conducting wire(s) to said external electrical stimulator and an earth electrode in contact with the skin.

2. Transcutaneous connecting device according to claim 1, characterised in that said external connecting means comprise external means (2,2a) connected to said subcutaneous conducting wire(s) (3,3a) and removable external means (4,30) connected to said external electrical stimulator.

3. Transcutaneous connecting device according to claim 1 or 2, characterised in that it is placed under the navicular furrow in a fold of the ear pinna.

4. Transcutaneous connecting device according to claim 3, characterised in that it comprises a subcutaneous conducting wire (3) covered with an insulating sheath, fixed to a ball (2) of conducting metal positioned on the surface of the ear pinna and covered with an insulating material on the part of its outer surface in contact with the ear pinna.

5. Transcutaneous connecting device according to claim 4, characterised in that the subcutaneous conducting wire (3) and the ball (2) are made of platinum-iridium.

6. Transcutaneous connecting device according to claim 4 or 5, characterised in that the subcutaneous conducting wire (3) and the surface of the ball (2) in contact with the ear pinna are insulated by means of Teflon® and Parylene®.

7. Transcutaneous connecting device according to any of the preceding claims, characterised in that the ball (2) is connected to the external stimulator by means of a metallic or metallized removable ear-piece (4) matching the contours of the pinna of the ear.

8. Transcutaneous connecting device according to claim 7, characterised in that the ball (2) is positioned by snapping into a cavity (5) formed in the removable ear-piece (4), of shape and dimensions corresponding essentially to those of the ball (2), the cavity being insulated from the ear-piece.

9. Transcutaneous connecting device according to claim 8, characterised in that the surface of the cavity (5) is connected to the external stimulator and constitutes the active pole, the metallic mass or the metallized surface of the removable ear-piece (4) affording earth contact with the skin of the pinna and of the auditory canal.

10. Transcutaneous connecting device according to claim 1, characterised in that it comprises an earring (2a) having on the one hand a post (10) connected to a subcutaneous assembly comprising at least one conducting wire (3a) the distal end of which is connected to the subcutaneous electrical conductor and the proximal end to the said post (10) and, on the other hand, a clasp (12) connected to the electrical stimulator outside the body, and in that the conducting wire (3a) is made of a biocompatible material.

11. Transcutaneous connecting device according to claim 4 or 10, characterised in that the conducting wire (3, 3a) is coiled.

12. Transcutaneous connecting device according to claim 11, characterised in that the conducting wire (3,3a) forms a spring.

13. Transcutaneous connecting device according to claim 12, characterised in that the conducting wire (3,3a) is made of Phynox®.

14. Transcutaneous connecting device according to claim 13, characterised in that the wire (3,3a) is covered with an insulating material, preferably Parylene®.

15. Transcutaneous connecting device according to claim 1, characterised in that it comprises three coiled subcutaneous conducting wires (3,3a) and constitute three helical springs which are interlaced with one another.

16. Transcutaneous connecting device according to claim 1, characterised in that it comprises three subcutaneous conducting wires (3,3a) braided together.

17. Transcutaneous connecting device according to claim 14, characterised in that the post (10) of the earring (2a) is insulated by means of a biocompatible plastic sheath (16).

18. Transcutaneous connecting device according to any one of claims 4 to 17, characterised in that the conducting wire (3,3a) is connected to the subcutaneous electrical conductor by means of at least two electrical connectors (20).

19. Transcutaneous connecting device according to claim 18, characterised in that each electrical connector (20) consists of a composite piece comprising a male part forming a pin (21) and a female part (22) forming a socket in which the male part (21) forming the pin of the other connector (20) engages.

20. Transcutaneous connecting device according to claim 10, characterised in that the earring (2a) is connected to the external stimulator by means of a magnet (24).

21. Transcutaneous connecting device according to claim 20, characterised in that the earring is connected to the external stimulator by means of a cylindrical magnet fixed to the earring, to which is applied a removable magnetic mass (30) of the same shape connected to the output of the stimulator.

22. Transcutaneous connecting device according to claim 21, characterised in that the removable magnetic mass (30) consists of a second magnet.

23. Transcutaneous connecting device the ear (sic) according to claim 23, characterised in that the magnet (24) is fixed in one of the branches (14) of the clasp of the earring by means of a conductive metallic sheath (25) fixed in the branch of the clasp by welding, in such a way that the edge (26) of the said conductive metallic sheath projects relative to the said magnet (24) in such a way as to receive the magnet (30), connected to the output of the external stimulator, the two magnets being oriented to attract each other upon connection.

24. Transcutaneous connecting device according to any one of the preceding claims, characterised in that it comprises several subcutaneous conducting wires (46) and in that the connection of the subcutaneous wires to the external stimulator is achieved by means of a connection assembly comprising a male connector (41) and a female conductor (42) held in the insertion position by means of at least one magnet (55,64), the said male (41) and female (42) connectors comprising respective polarizing slot elements (54,65).

## Patentansprüche

1. Transkutane Verbindungseinrichtung zur Verbindung eines außerhalb des Organismus befindlichen elektrischen Stimulators mit einem subkutanen elektrischen Leiter, der mit einer Elektrode verbunden ist, dadurch **gekennzeichnet**, daß sie in der Ohrmuschel befestigt ist und wenigstens einen von einem isolierenden Mantel umgebenen subkutanen Leitungsdraht (3, 3a), dessen distales Ende mit dem subkutanen elektrischen Leiter verbunden ist, äußere Verbindungsmittel (2, 4-2a, 30) der Ohrmuschel zur Verbindung des proximalen Endes des subkutanen Leitungsdrahtes oder der subkutanen Leitungsdrähte mit dem externen elektrischen Stimulator und eine mit der Haut in Kontakt stehende Masseelektrode aufweist.

2. Transkutane Verbindungseinrichtung nach Anspruch 1, dadurch **gekennzeichnet**, daß die äußeren Verbindungsmittel äußere Verbindungsmittel (2, 2a), die mit dem subkutanen Leitungsdraht (3, 3a) oder den subkutanen Leitungsdrähten verbunden sind, und lösbare äußere Mittel (4, 30) umfassen, die mit dem externen elektrischen Stimulator verbunden sind.

3. Transkutane Verbindungseinrichtung nach Anspruch 1 oder 2, dadurch **gekennzeichnet**, daß sie unterhalb der Dreiecksgrube in einer Falte der Ohrmuschel angebracht ist.

4. Transkutane Verbindungseinrichtung nach Anspruch 3, dadurch **gekennzeichnet**, daß sie einen von einem isolierenden Mantel umgebenen subkutanen Leitungsdraht (3) aufweist, der an einer Kugel (2) aus leitfähigem Metall befestigt ist, die an der Oberfläche der Ohrmuschel angeordnet und auf dem mit der Ohrmuschel in Berührung stehenden Teil ihrer äußeren Oberfläche mit einem isolierenden Material beschichtet ist.

5. Transkutane Verbindungseinrichtung nach Anspruch 4, dadurch **gekennzeichnet**, daß der subkutane Leitungsdraht (3) und die Kugel (2) aus Platin-Iridium bestehen.

6. Transkutane Verbindungseinrichtung nach Anspruch 4 oder 5, dadurch **gekennzeichnet**, daß der subkutane Leitungsdraht (3) und die mit der Ohrmuschel in Berührung stehende Oberfläche der Kugel (2) durch Teflon® und Parylène® isoliert sind.

7. Transkutane Verbindungseinrichtung nach einem der vorstehenden Ansprüche, dadurch **gekennzeichnet**, daß die Kugel (2) mit dem externen Stimulator durch einen metallischen oder metallisierten lösbaren Stöpsel (4) verbunden ist, der an das Relief der Ohrmuschel angepaßt ist.

8. Transkutane Verbindungseinrichtung nach Anspruch 7, dadurch **gekennzeichnet**, daß die Kugel (2) in einem in dem lösbaren Stöpsel (4) ausgebildeten Napf (5) verrastet ist, dessen Form und Abmessungen im wesentlichen denen der Kugel (2) entsprechen und der von dem Stöpsel isoliert ist.

9. Transkutane Verbindungseinrichtung nach Anspruch 8, dadurch **gekennzeichnet**, daß die Oberfläche des Napfes (5) mit dem externen Stimulator verbunden ist und den aktiven Pol bildet, wobei die metallische Masse oder die metallisierte Oberfläche des lösbaren Stöpsels (4) einen Massekontakt mit der Haut der Ohrmuschel und des Gehörgangs sicherstellt.

10. Transkutane Verbindungseinrichtung nach Anspruch 1, dadurch **gekennzeichnet**, daß sie einen Ohrstecker (2a) aufweist, der einerseits einen Dorn (10), der mit einer subkutanen Einheit verbunden ist, die wenigstens einen Leitungsdraht (3a), dessen distales Ende mit dem subkutanen elektrischen Leiter verbunden ist und dessen proximales Ende mit dem Dorn (10) verbunden ist, aufweist, und andererseits eine Schließe (12) aufweist, die mit dem elektrischen Stimulator außerhalb des Organismus verbunden ist, und daß der Leitungsdraht (3a) aus einem biokompatiblen Material besteht.

11. Transkutane Verbindungseinrichtung nach Anspruch 4 oder 10, dadurch **gekennzeichnet**, daß der Leitungsdraht (3, 3a) verdrillt ist.

12. Transkutane Verbindungseinrichtung nach Anspruch 11, dadurch **gekennzeichnet**, daß der Leitungsdraht (3, 3a) eine Feder bildet.

13. Transkutane Verbindungseinrichtung nach Anspruch 12, dadurch **gekennzeichnet**, daß der Leitungsdraht (3, 3a) aus Phynox® besteht.

14. Transkutane Verbindungseinrichtung nach Anspruch 13, dadurch **gekennzeichnet**, daß der Draht (3, 3a) von einem isolierenden Material, vorzugsweise Parylène® umhüllt ist.

15. Transkutane Verbindungseinrichtung nach Anspruch 1, dadurch **gekennzeichnet**, daß sie drei verdrillte subkutane Leitungsdrähte (3, 3a) aufweist, die drei miteinander verzahnte Schraubenfedern bilden.

16. Transkutane Verbindungseinrichtung nach Anspruch 1, dadurch **gekennzeichnet**, daß sie drei miteinander verflochtene subkutane Leitungsdrähte (3, 3a) aufweist.

17. Transkutane Verbindungseinrichtung nach Anspruch 14, dadurch **gekennzeichnet**, daß der Dorn (10) des Ohrsteckers (2a) durch eine biokompatible Plastikhülse (16) isoliert ist.

18. Transkutane Verbindungseinrichtung nach einem der Ansprüche 4 bis 17, dadurch **gekennzeichnet**, daß der Leitungsdraht (3, 3a) mit dem subkutanen elektrischen Leiter durch mindestens zwei elektrische Verbinder (20) verbunden ist.

19. Transkutane Verbindungseinrichtung nach Anspruch 18, dadurch **gekennzeichnet**, daß Jeder elektrische Verbinder (20) aus einem Verbundteil besteht, das einen Steckerteil (21) und einen Buchsenteil (22) aufweist, in den der Steckerteil (21) des anderen Verbinders (20) eingreift.

20. Transkutane Verbindungseinrichtung nach Anspruch 10, dadurch **gekennzeichnet**, daß der Ohrstecker (2a) mit dem externen Stimulator mit Hilfe eines Magneten (24] verbunden ist.

21. Transkutane Verbindungseinrichtung nach Anspruch 20, dadurch **gekennzeichnet**, daß der Ohrstecker mit dem externen Stimulator mit Hilfe eines Magneten mit zylindrischer Form verbunden ist, der an dem Ohrstecker befestigt ist und an den sich ein lösbarer magnetischer Körper (30) von derselben Form anlegt, der mit dem Ausgang des Stimulators verbunden ist.

22. Transkutane Verbindungseinrichtung nach Anspruch 21, dadurch **gekennzeichnet**, daß der lösbare magnetische Körper (30) aus einem zweiten Magneten besteht.

23. Transkutane Verbindungseinrichtung nach Anspruch 22, dadurch **gekennzeichnet**, daß der Magnet (24) an einem der Arme (14) der Schließe des Ohrsteckers mit Hilfe einer leitenden metallischen Hülse (25) verbunden ist, die in dem Arm der Schließe so durch Löten oder Schweißen befestigt ist, daß der Rand (26) der leitenden metallischen Hülse über den Magneten (24) hinausragt, so daß er den mit dem Ausgang des externen Stimulators verbundenen Magneten (30) aufnimmt, wobei die beiden Magnete so orientiert sind, daß sie sich bei der Verbindung gegenseitig anziehen.

24. Transkutane Verbindungseinrichtung nach einem der vorstehenden Ansprüche, dadurch **gekennzeichnet**, daß sie mehrere subkutane Leitungsdrähte (46) aufweist und daß die Verbindung der subkutanen Leitungdrähte mit dem externen Stimulator durch eine Verbindungseinheit mit einem Stecker (41) und einer Buchse (42) erfolgt, die durch wenigstens einen Magneten (55, 64) in Steckverbindung gehalten werden, wobei der Stecker (41) und die Buchse (42) jeweilige Unverwechselbarkeitseinrichtungen (64, 65) aufweisen.
